# EUROPEAN PATENT APPLICATION

(11) **EP 4 112 095 A1**
(43) Date of publication of application: **04.01.2023**
(21) Application number: 21761473.4
(22) Date of filing: 05.03.2021
(51) Int. Cl.: A61M 5/142, A61M 5/00, A61M 5/14

(54) **COVER BODY AND DEVICE FOR INJECTING MEDICAL LIQUID COMPRISING SAME**

(30) Priority: 28.02.2020 KR 20200025518
(71) Applicant: Eoflow Co., Ltd., Bundang-gu, Seongnam-si Gyeonggi-do 13605 (KR)
(72) Inventor: KIM, Seung Ha, Goyang-si Gyeonggi-do 10567 (KR)
(74) Representative: Mewburn Ellis LLP
(86) International application number: PCT/KR2021/002726
(87) International publication number: WO 2021/172972

(57) **Abstract**

A liquid medication injection device includes a housing having an opening on one side thereof, a sealing part disposed inside the housing and covering the opening, a storage container disposed inside the housing and storing liquid medication, and an injection needle which is connected to the storage container and through which the liquid medication is injected via the sealing part.

## Description

The present disclosure relates to a device for microinjecting a liquid medication such as insulin and a cover usable therefor.

### BACKGROUND ART

Diabetes is a disease based on metabolic abnormalities occurring when insulin, one of the hormones secreted by the pancreas, lacks. Diabetic patients can use, as one of the active methods, the method of injecting insulin into the body. An insulin injection device may be used to adjust the amount of insulin injected into the body to be appropriate for changes in blood sugar of a patient.

A liquid medication injection device such as an insulin injection device is used semi-permanently by re-filling with a liquid medication. Accordingly, it is necessary to prevent foreign substances (e.g., air) other than the liquid medication from being injected through the injection needle or to prevent the device from being contaminated.

### DESCRIPTION OF EMBODIMENTS

### TECHNICAL PROBLEM

Embodiments of the present disclosure relate to a cover for preventing contamination of the inside of a device and a liquid medication injection device including the same.

### TECHNICAL SOLUTION TO PROBLEM

An embodiment of the present disclosure provides a liquid medication injection device including a housing having an opening on one side thereof, a sealing part disposed inside the housing and covering the opening, a storage container disposed inside the housing and storing liquid medication, and an injection needle which is connected to the storage container and through which the liquid medication is injected via the sealing part.

### ADVANTAGEOUS EFFECTS OF DISCLOSURE

A liquid medication injection device according to embodiments of the present disclosure includes a cover which is capable of preventing the backflow of air during a priming operation of removing air from an injection needle, and is capable of preventing contamination of the device. These effects do not limit the scope of the present disclosure.

### BRIEF DESCRIPTION OF DRAWINGS

FIG. 1 shows a perspective view schematically showing a liquid medication injection device according to an embodiment of the present disclosure.
FIG. 2 shows an exploded perspective view of FIG. 1.
FIG. 3 shows a schematic, perspective view of a part of a main body according to an embodiment of the present disclosure.
FIG. 4 shows an exploded schematic, perspective view of a cover according to an embodiment of the present disclosure.
FIG. 5 shows an internal perspective view of the main body according to an embodiment of the present disclosure to describe the inside of the main body.
FIG. 6 shows the cover and the main body before coupling, and FIG. 7 illustrates the cover and the main body after coupling.

### BEST MODE

A liquid medication injection device includes a housing having an opening on one side thereof, a sealing part disposed inside the housing and covering the opening, a storage container disposed inside the housing and storing liquid medication, and an injection needle which is connected to the storage container and through which the liquid medication is injected via the sealing part.

In one embodiment of the present disclosure, the sealing part may have a shape that is concave toward the inside of the housing.

In one embodiment of the present disclosure, the sealing part may include a concave portion formed to be concave toward the inside of the housing, and an edge portion surrounding the concave portion.

In one embodiment of the present disclosure, the housing may further include a fixing part which has an opening exposing the concave portion of the sealing part and fixes the edge portion of the sealing part.

In one embodiment of the present disclosure, a cover positioned on the opposite side of the sealing part with the opening therebetween, may be further included.

In one embodiment of the present disclosure, the cover may further include a frame having a hole corresponding to the opening of the housing.

In one embodiment of the present disclosure, the frame may further include a sleeve that protrudes to the outside and engages with the opening.

In one embodiment of the present disclosure, the cover may further include a cover part which is positioned on the opposite side of the sleeve with the hole therebetween and which covers the hole.

In one embodiment of the present disclosure, the cover part may include an air-permeable material.

According to one embodiment of the present disclosure, the cover for a liquid medication injection device includes a frame having a hole and a cover part coupled to the frame, wherein the cover part includes an air-permeable material.

In one embodiment of the present disclosure, the frame may further include a sleeve that protrudes to the outside and is coupled to the opening of the liquid medication injection device, and the cover part is located on the opposite side of the sleeve with the hole therebetween and covers the hole.

Other aspects, features and advantages than those described above will become apparent from the following detailed description, claims and drawings for embodying the disclosure.

### MODE OF DISCLOSURE

The present disclosure can be subjected to various transformations and can have various embodiments, and accordingly, specific embodiments are illustrated in the drawings and described in detail. However, this is not intended to limit the present disclosure to specific embodiments, and should be understood to include all modifications, equivalents, and substitutes included in the concept and scope of the present disclosure. In describing the present disclosure, if it is determined that a detailed description of a related known technology may make the gist of the present disclosure unclear, the detailed description thereof will be omitted.

Terms such as first, second, etc. may be used to describe various elements, but the elements should not be limited by the terms. The terms are used only for the purpose of distinguishing one component from another.

The terms used in the present application are only used to describe specific embodiments, and are not intended to limit the present disclosure. The singular expression includes the plural expression unless the context clearly dictates otherwise. In the present application, terms such as "comprise" or "have" are intended to designate that a feature, a number, a step, an operation, a component, a part, or a combination thereof described in the specification exists, and it should be understood that it does not preclude the possibility of addition or existence of one or more other features, numbers, steps, operations, components, parts, or combinations thereof.

Hereinafter, embodiments of the present disclosure will be described in detail with reference to the accompanying drawings. Here, in the description with reference to the accompanying drawings, the same or corresponding components are given the same reference numerals, and the redundant description thereof will be omitted.

In addition, in describing various embodiments of the present disclosure, each embodiment does not have to be independently interpreted or practiced. And, it should be understood that the technical ideas described in each embodiment can be interpreted or implemented in combination in other embodiments described individually.

Hereinafter, an embodiment of the present disclosure will be described with reference to the accompanying drawings.

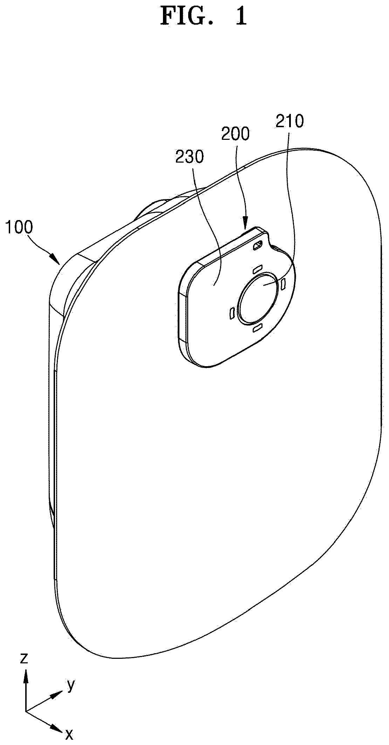

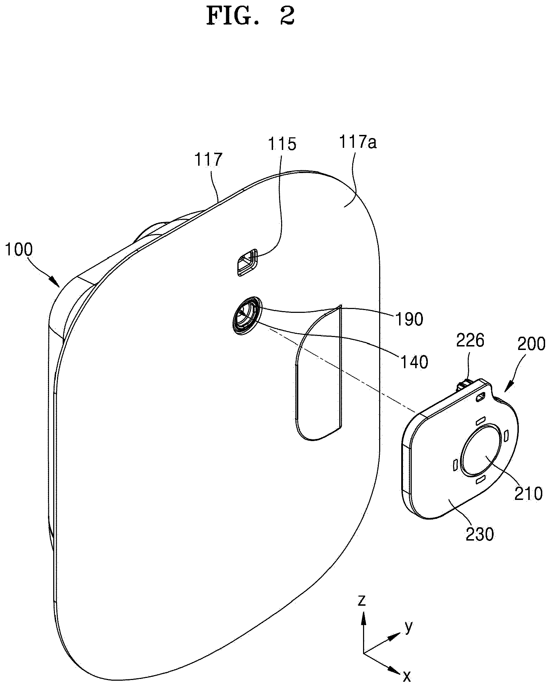

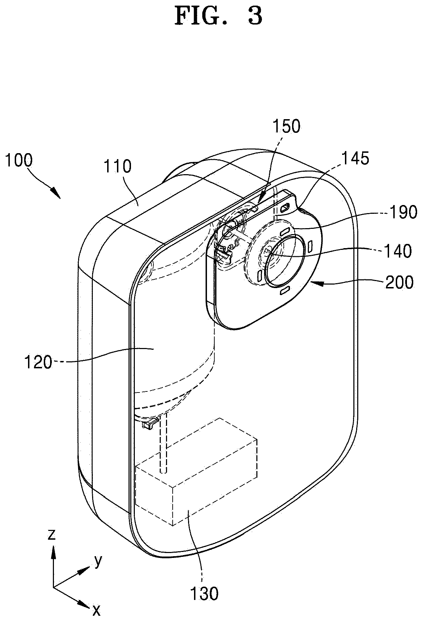

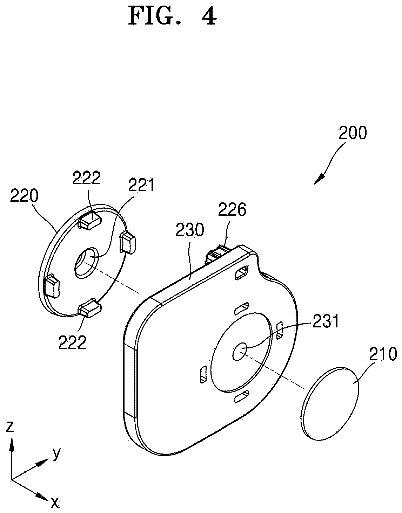

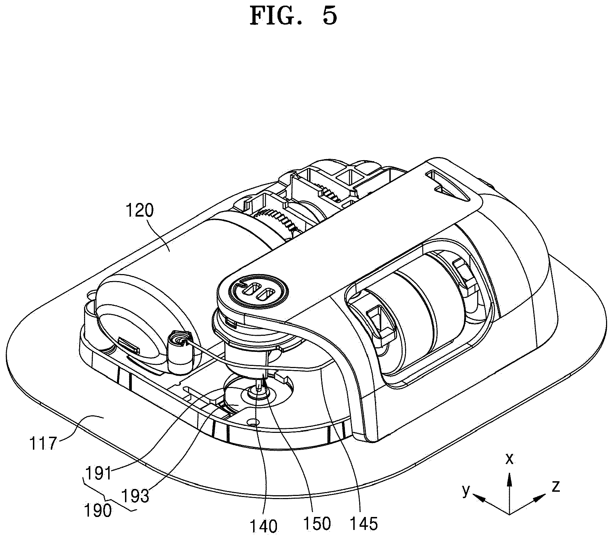

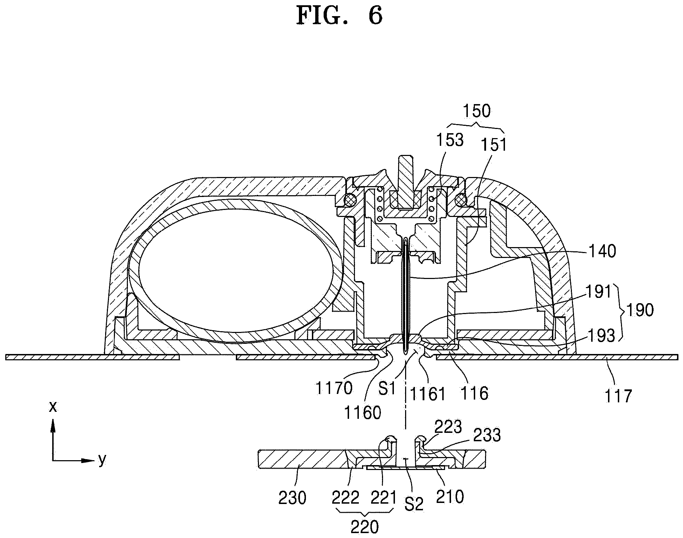

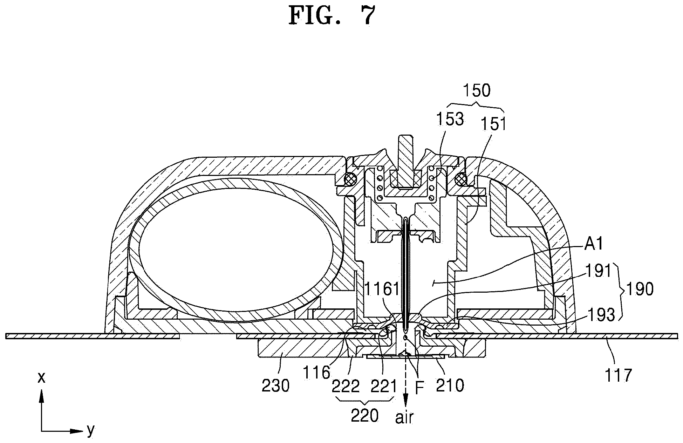

FIG. 1 shows a perspective view schematically showing a liquid medication injection device according to an embodiment of the present disclosure. FIG. 2 shows an exploded perspective view of FIG. 1. FIG. 3 shows a schematic, perspective view of a part of a main body according to an embodiment of the present disclosure. In FIG. 3, the patch plate 117 is omitted for convenience of description. FIG. 4 shows an exploded schematic, perspective view of a cover according to an embodiment of the present disclosure. FIG. 5 shows an internal perspective view of the main body according to an embodiment of the present disclosure to describe the inside of the main body. FIG. 6 illustrates the cover and the main body before coupling, and FIG. 7 illustrates the cover and the main body after coupling.

Referring to FIGS. 1 to 7, a liquid medication injection device 1 includes a main body 100 and a cover 200. The main body 100 includes a housing 110 having an opening 1160 on one side thereof, a sealing part 190 covering the opening 1160, a storage container 120 positioned inside the housing 110, and an injection needle 140 connected to the storage container 120.

The housing 110 may include a material such as plastic or a metallic material, and the opening (1160, see FIG. 6) may be formed on one side of the housing 110. The housing 110 may include a patch plate 117 disposed on one side of the housing 110 having the opening 1160. The patch plate 117 may have an adhesive surface 117A capable of attaching the liquid medication injection device 1 to the patient's skin surface. The adhesive surface 117A of the patch plate 117 is protected by a release paper (not shown) and, when there is a need for the liquid medication injection device 1 to be attached on the skin surface of the patient, the release paper may be removed for use. The patch plate 117 may have an opening 1170 corresponding to the opening 1160 of the housing 110. Hereinafter, for convenience of description, the opening formed in the housing 110 will be referred to as a first opening, and the opening formed in the patch plate 117 will be referred to as a second opening.

The housing 110 may further include a fixing part 116 having a first opening 1160 and constituting one side of the housing 110. The fixing part 116 may fix the sealing part 190 to the inside of the housing 110 while exposing a portion of the sealing part 190 to be described later through the first opening 1160.

The sealing part 190 is disposed inside the housing 110 and covers the first opening 1160. The sealing part 190 may cover the first opening 1160 for exposing the injection needle 140, to block the inner space of the housing 110 from the outside.

The sealing part 190 may have a shape that is concave toward the inside of the housing 110. Specifically, the sealing part 190 may include a concave portion 191 formed to be concave toward the inside of the housing 110, and an edge portion 193 surrounding the concave portion 191. The concave portion 191 may be disposed in the center of the sealing part 190, and the edge portion 193 may be disposed at an edge of the sealing part 190.

In one embodiment, the concave portion 191 and the edge portion 193 are integrally molded, and the concave portion 191 and the edge portion 193 may include an identical material. At this time, the fixing part 116 of the housing 110 may fix the sealing part 190 to the inside the housing 110 by exposing the concave portion 191 of the sealing part 190 through the first opening 1160 and simultaneously, supporting the edge portion 193 of the sealing part 190. Meanwhile, a cross-sectional thickness of the concave portion 191 may be greater than a cross-sectional thickness of the edge portion 193. As a result, the concave portion 191 may be fitted into the opening of the body part 151 of an injection needle assembly 150 to be described later.

The sealing part 190 may form a first space S1 in the first opening 1160 through the concave portion 191. The first space S1 is a space separated from the interior of the housing 110, and the injection needle 140 may be placed in the first space S1 while a portion thereof is exposed to the first space S1 before liquid medication is injected to the patient.

The sealing part 190 may include a material that allows the injection needle 140 to penetrate therethrough while maintaining tightness with the housing 110. For example, the sealing part 190 may include a polymer. An example of the polymer may be natural rubber or synthetic rubber, or a silicone-based polymer. The silicone-based polymer may include polydimethylsiloxane (PDMS), hexamethyldisiloxane (HMDSO), or the like. In another embodiment, an example of the elastic material may be polyurethane or polyurethane acrylate.

The storage container 120 may store liquid medication an amount corresponding to such an amount that can be injected several to tens of times. In one embodiment, the liquid medication injection device 1 may be configured to be disposable. However, the present disclosure is not limited thereto. For example, the liquid medication injection device 1 may be configured for a multi-use. In this case, when the liquid medication stored in the storage container 120 is used up, a new liquid medication is refilled in the storage container 120.

A pump 130 is a small pump, for example, a micro-pump, and may pump a certain amount of a liquid medication. In one embodiment, the pump 130 may pump liquid medication of several tens of nanoliters to several microliters, and may generate a flow rate of several microliters to several tens of microliters per minute, but the present disclosure is not limited thereto. The pump 130 may pump liquid medication directly, or may be connected to other driving means to indirectly move liquid medication.

The injection needle 140 may be connected to the storage container 120 through a tube 145. liquid medication in the storage container 120 may be transferred to the injection needle 140 through the tube 145 by the pump 130. When a button (not shown) provided in the main body 100 is pressed, the injection needle 140 protrudes to the outside through the opening 1160 of the main body 100 according to a control operation of a controller (not shown) to be in a state in which liquid medication is injectable to the patient.

In one embodiment, the injection needle 140 may be coupled to the injection needle assembly 150 to protrude to the outside or to be restored to an initial position, according to a control operation of a controller (not shown). In this case, the injection needle assembly 150 may include a driving unit (not shown) connected to the injection needle 140 and the body part 151 that forms the exterior of the injection needle assembly 150 by arranging the injection needle 140 therein. An opening, through which the injection needle 140 passes, may be formed in the body part 151, and the concave portion 191 of the sealing part 190 may be inserted into the opening of the body part 151 to increase the sealing force. In addition, the edge portion 193 of the sealing part 190 may be disposed and fixed between the body part 151 and the fixing part 116.

In addition, the injection needle assembly 150 may further include an elastic part 153 that provides a restoring force to the injection needle 140. For example, the injection needle 140 may receive a driving force from a driving unit (not shown) and move to protrude to the outside. In addition, the injection needle 140 may be restored to the initial position thereof by the elastic part 153 when the driving force is removed by the control operation of the controller (not shown).

The cover 200 may cover the first opening 1160 of the main body 100. The cover 200 may be fixedly coupled to a hole 115 formed on one side of the housing 110 by using a protrusion 226 provided in the frame 230. The cover 200 may be positioned opposite the sealing part 190 with the first opening 1160 therebetween. The cover 200 may include a frame 230 having a hole and a cover part 210 covering the hole, and may further include an auxiliary frame 220 coupled to the frame 230.

The cover part 210 may be disposed on one side of the frame 230 to cover a hole 231. The cover part 210 may include an air-permeable material. The cover part 210 may include a material that transmits air discharged from the injection needle 140, but does not transmit liquid medication. For example, the cover part 210 may include polytetrafluoroethylene (PTFE), expanded PTFE (ePTFE), Tyvek, and Kynar polyvinylidene fluoride (PVDF), etc.

The frame 230 is provided at a position corresponding to the first opening 1160 of the housing 110 and may have the hole 231 penetrating the frame 230. In addition, the frame 230 may further include a sleeve 233 positioned on one side facing the main body 100 and protruding to the outside to engage with the opening of the main body 100. By being coupled to the opening 1160 of the main body 100, the sleeve 233 may fix the cover 200 to the main body 100. In addition, together therewith, the protrusion 226 is formed on one side of the frame 230 to allow the cover 200 to be stably fixed on the main body 100. The sleeve 233 protrudes from the frame 230 in the x direction.

The auxiliary frame 220 may have a second hole 231 overlapping the hole 231 (hereinafter, referred to as the first hole) of the frame 230, and may include a leg part 222 protruding toward the frame 230. The auxiliary frame 220 may be coupled to the frame 230 using one or more leg portions 222, and may include a second sleeve 223 which surrounds the sleeve 233 (hereinafter, referred to as the first sleeve) of the frame 230 and supports the first sleeve 233. The auxiliary frame 220 may include the same material as the frame 230, but the present disclosure is not limited thereto. In an embodiment, the auxiliary frame 220 may include the same material as the sealing part 190, and may provide an increase in fixing force and tightness while the cover 200 is coupled to the main body 100.

The auxiliary frame 220 may include a first bent part 221 protruding toward the outside at one end of the second sleeve 223. The auxiliary frame 220 may be more stably fixed using the first bent portion 221 when the same is inserted into the main body 100. To this end, the housing 110 of the main body 100 may further include a second bent portion 1161 extending in the central direction of the first opening 1160 and formed at one end of the first opening 1160. The first bent portion 221 of the auxiliary frame 220 may be coupled to the second bent portion 1161 of the first opening 1160 to fix the cover 200 on the main body 100.

The cover 200 may form a second space S2 through the first hole 231 formed in the frame 230. During the liquid medication injection operation, the cover 200 is separated from the main body 100, and as described above, according to the operation of the pump 130, the liquid medication in the storage container 120 is injected into the patient through the injection needle 140.

When the injection needle 140 contains air before the operation of injecting the liquid medication, an operation of removing the air (priming operation) is performed. This operation may be performed manually by the user or may be automatically made by the pump 130 driven according to a signal from the control unit, through which the air in the injection needle 140 may be, as shown in FIG. 7, discharged. The discharged air may be discharged to the outside through the second space S2 and then through the cover part 210, which includes an air-permeable material. In the case where the cover part 210 does not transmit the air, even when the air is discharged from the injection needle 140, the second space S2 may not have a space for housing the discharged air and thus, the air may back-flow into the injection needle 140, and further, injected into the patient. However, according to embodiments of the present disclosure, the air discharged from the injection needle 140 may be discharged directly to the outside by the sealing part 190 made of an air-permeable material. Accordingly, the issue described above may be addressed.

During the priming operation, a small amount of liquid medication F may be discharged together with air. A small amount of liquid medication F may be located in the second space S2 along the discharge direction (x direction) of the liquid medication. At this time, since the main body 100 covers the first opening 1160 by the sealing part 190, the contamination of the inside of the main body 100 by a small amount of liquid medication F may be prevented.

As described above, the cover and the liquid medication injection device having the same, according to embodiments of the present disclosure, can prevent the backflow of air during the priming operation, which is an operation to remove air from the injection needle, and prevent contamination of the device by the sealing part.

As such, the present disclosure has been described with reference to the embodiments illustrated in the drawings, which are merely examples, and those skilled in the art would understand that various modifications and equivalents thereof or thereto can be made. Therefore, the true technical protection scope of the present disclosure should be determined by the technical spirit of the appended claims.

### [INDUSTRIAL APPLICABILITY]

According to one embodiment of the present disclosure, a liquid medication injection device is provided. In addition, embodiments of the present disclosure can be applied to industrially used insulin administration devices.

## Claims

1. A liquid medication injection device comprising:
a housing having an opening on one side thereof;
a sealing part disposed inside the housing and covering the opening;
a storage container disposed inside the housing and storing liquid medication; and
an injection needle which is connected to the storage container and through which the liquid medication is injected via the sealing part.

2. The liquid medication injection device of claim 1, wherein the sealing part has a shape that is concave toward the inside of the housing.

3. The liquid medication injection device of claim 2, wherein the sealing part has a concave portion that is concave toward the inside of the housing, and an edge portion surrounding the concave portion.

4. The liquid medication injection device of claim 3, wherein the housing further comprises a fixing part which has an opening exposing the concave portion of the sealing part, and fixes the edge portion of the sealing part.

5. The liquid medication injection device of claim 1, further comprising
a cover positioned on the opposite side of the sealing part with the opening therebetween.

6. The liquid medication injection device of claim 5, wherein the cover further comprises a frame having a hole corresponding to the opening of the housing.

7. The liquid medication injection device of claim 6, wherein the frame further comprises a sleeve protruding to the outside and coupled to the opening.

8. The liquid medication injection device of claim 7, wherein the cover further comprises a cover part which is positioned on the opposite side of the sleeve with the hole therebetween, and covers the hole.

9. The liquid medication injection device of claim 8, wherein the cover part includes an air-permeable material.

10. A cover for a liquid medication injection device, the cover comprising:
a frame having a hole; and
a cover part coupled to the frame,
wherein the cover part includes an air-permeable material.

11. The cover of claim 10, wherein
the frame further includes a sleeve that protrudes to the outside and engages with the opening of the liquid medication injection device, and
the cover part is positioned on the opposite side of the sleeve with the hole therebetween to cover the hole.
